# EUROPEAN PATENT SPECIFICATION

(11) **EP 3 977 956 B1**
(45) Date of publication and mention of the grant of the patent: **27.08.2025**
(21) Application number: 21200450.1
(22) Date of filing: 01.10.2021
(51) Int. Cl.: A61B 34/30, A61B 34/37, A61B 90/00, A61B 18/14, A61B 17/00, A61B 18/00

(54) **FINE DISSECTION END EFFECTOR ASSEMBLY**
ENDEFFEKTORANORDNUNG FÜR FEINDISSEKTION
ENSEMBLE EFFECTEUR TERMINAL DE DISSECTION FINE

(30) Priority: 02.10.2020 US 202017061846
(43) Date of publication of application: 06.04.2022
(73) Proprietor: Covidien LP, Mansfield, MA 02048 (US)
(72) Inventor: NETZEL, Kenneth, E, Boulder, CO Colorado 80301 (US)
(74) Representative: Maschio & Soames IP Ltd

(56) References cited:
- US-A1- 2012 215 234
- US-A1- 2014 257 284
- US-A1- 2015 245 866

## Description

### FIELD

The present disclosure relates to surgical instruments and, more particularly, to electrosurgical instruments for treating and dissecting tissue.

### BACKGROUND

A surgical forceps is a pliers-like instrument that relies on mechanical action between its jaw members to grasp, clamp, and constrict tissue. Electrosurgical forceps utilize both mechanical clamping action and energy to heat tissue to treat, e.g., coagulate, cauterize, dissect or seal, tissue. Typically, prior to tissue being treated, a surgeon must dissect portions of the tissue to orient the tissue for treatment. Examples of dissection include "blunt" dissection wherein the end of the end effector is used to bluntly separate tissue. Other examples include "poke and spread" dissection wherein the tissue is engaged and then the jaw members of the end effector are opened to spread the tissue. Still in other instances, a surgeon may have to finely dissect tissue in a grasp and pull manner.

Once the tissue is treated, the surgeon may have to accurately sever the treated tissue or further dissect portions thereof. Accordingly, many electrosurgical forceps are designed to incorporate a knife that is advanced between the jaw members to cut the treated tissue. As an alternative to a mechanical knife, an energy-based tissue cutting element may be provided to cut the treated tissue using energy, e.g., thermal, electrosurgical, ultrasonic, light, or other suitable energy.

US 2014/257284 A1 describes an end effector assembly with first and a second jaws having a U-shaped proximal flanges to support a pivot and a pivoting bar therein.

### SUMMARY OF THE INVENTION

The invention is defined by the appended independent claim. Further embodiments of the invention are illustrated in the dependent claims.

### SUMMARY OF THE DISCLOSURE

As used herein, the term "distal" refers to the portion that is being described which is further from a user, while the term "proximal" refers to the portion that is being described which is closer to a user. Further, to the extent consistent, any or all of the aspects detailed herein may be used in conjunction with any or all of the other aspects detailed herein.

Provided in accordance with aspects of the present disclosure is an electrosurgical instrument that includes a housing having a handle and an elongated shaft extending therefrom supporting an end effector assembly at a distal end thereof. The end effector assembly includes a first jaw member having a jaw housing supporting an electrically conductive tissue engaging surface thereon. The jaw housing has a U-shaped proximal flange including opposing sides defining a cuff therebetween. Each side has a cradle defined therein configured to secure a pivot bar therein. The proximal flange of the first jaw member is configured to operably support a pivot therein.

The end effector assembly also includes a second jaw member having a jaw housing supporting an electrically conductive tissue engaging surface thereon in opposition to the tissue engaging surface of the first jaw member. The jaw housing includes a U-shaped proximal flange having opposing sides defining a cuff therebetween configured to receive the proximal flange of the first jaw member. Each side of the second proximal flange includes a cradle defined therein configured to operably support the pivot and the proximal flange is configured to operably engage the distal end of the elongated shaft.

A drive tube is selectively translatable within the elongated shaft and includes a drive member disposed at a distal end thereof operably secured to the pivot bar. Actuation of the handle translates the drive member which, in turn, translates the pivot bar to rotate the first jaw member relative to the second jaw member about the pivot.

In aspects according to the present disclosure, the pivot bar is welded to the cradle of each side of the proximal flange of the first jaw member. In other aspects according to the present disclosure, the first jaw member moves relative the second jaw member upon actuation of the drive member.

In aspects according to the present disclosure, the second jaw member defines a longitudinal axis therethrough and the pivot is disposed on one side of the longitudinal axis and the pivot bar is offset from the pivot on the other side of the longitudinal axis. In other aspects according to the present disclosure, the cradle of the second jaw member is defined in an upper edge of the proximal flange thereof maximizing the offset between the pivot and the pivot bar increasing mechanical advantage therebetween. In yet other aspects according to the present disclosure, the offset between the pivot and the pivot bar provides a substantially constant closure force between the first and second jaw members through the range of motion therebetween. In still other aspects according to the present disclosure, the offset between the pivot and the pivot bar provides a substantially constant opening and closing force between the first and second jaw members through the range of motion therebetween.

In aspects according to the present disclosure, the elongated shaft defines an outer periphery and wherein the proximal flanges of the first and second jaw members remain inside the outer periphery during the range of motion between jaw members.

Provided in accordance with aspects of the present disclosure is an end effector assembly for an electrosurgical instrument that includes a first jaw member having a jaw housing supporting an electrically conductive tissue engaging surface thereon. The jaw housing includes a U-shaped proximal flange having opposing sides defining a cuff therebetween. Each side includes a cradle defined therein configured to secure a pivot bar therein and the proximal flange is configured to operably support a pivot therein.

The end effector assembly includes a second jaw member having a jaw housing supporting an electrically conductive tissue engaging surface thereon in opposition to the tissue engaging surface of the first jaw member. The jaw housing includes a U-shaped proximal flange having opposing sides defining a cuff therebetween configured to receive the proximal flange of the first jaw member. Each side of the second proximal flange includes a cradle defined therein configured to operably support the pivot. A drive member is operably secured to the pivot bar such that actuation the drive member translates the pivot bar to rotate the first jaw member relative to the second jaw member about the pivot.

In aspects according to the present disclosure, the pivot bar is welded to the cradle of each side of the proximal flange of the first jaw member. In other aspects according to the present disclosure, the first jaw member moves relative the second jaw member upon actuation of the drive member.

In aspects according to the present disclosure, the second jaw member defines a longitudinal axis therethrough and the pivot is disposed on one side of the longitudinal axis and the pivot bar is offset from the pivot on the other side of the longitudinal axis. In other aspects according to the present disclosure, the cradle of the second jaw member is defined in an upper edge of the proximal flange thereof maximizing the offset between the pivot and the pivot bar increasing mechanical advantage therebetween. In still other aspects according to the present disclosure, the offset between the pivot and the pivot bar provides a substantially constant closure force between the first and second jaw members through the range of motion therebetween. In yet other aspects according to the present disclosure, the offset between the pivot and the pivot bar provides a substantially constant opening and closing force between the first and second jaw members through the range of motion therebetween.

### BRIEF DESCRIPTION OF DRAWINGS

The above and other aspects and features of the present disclosure will become more apparent in view of the following detailed description when taken in conjunction with the accompanying drawings wherein like reference numerals identify similar or identical elements.
FIG. 1 is a perspective view of a shaft-based electrosurgical forceps provided in accordance with the present disclosure shown connected to an electrosurgical generator;
FIG. 2 is a schematic illustration of a robotic surgical instrument provided in accordance with the present disclosure;
FIG. 3A is a side, perspective view of an end effector assembly having first and second jaw members disposed in a spaced-apart position; and
FIG. 3B is a side, perspective view of the end effector assembly of FIG. 3A shown with a housing of the second jaw member removed.

### DETAILED DESCRIPTION

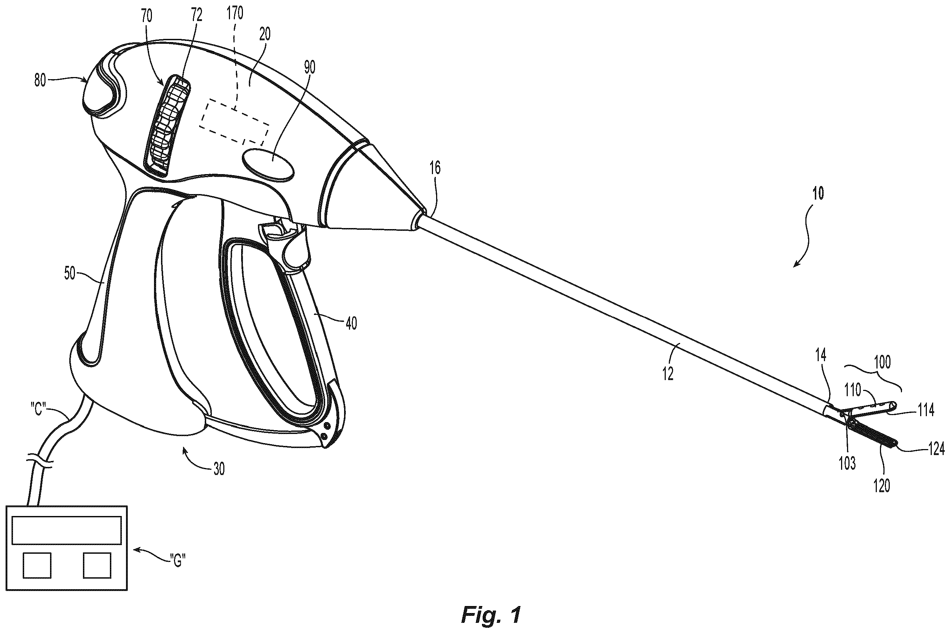

Referring to FIG. 1, a shaft-based electrosurgical forceps provided in accordance with the present disclosure is shown generally identified by reference numeral 10. Aspects and features of forceps 10 not germane to the understanding of the present disclosure are omitted to avoid obscuring the aspects and features of the present disclosure in unnecessary detail.

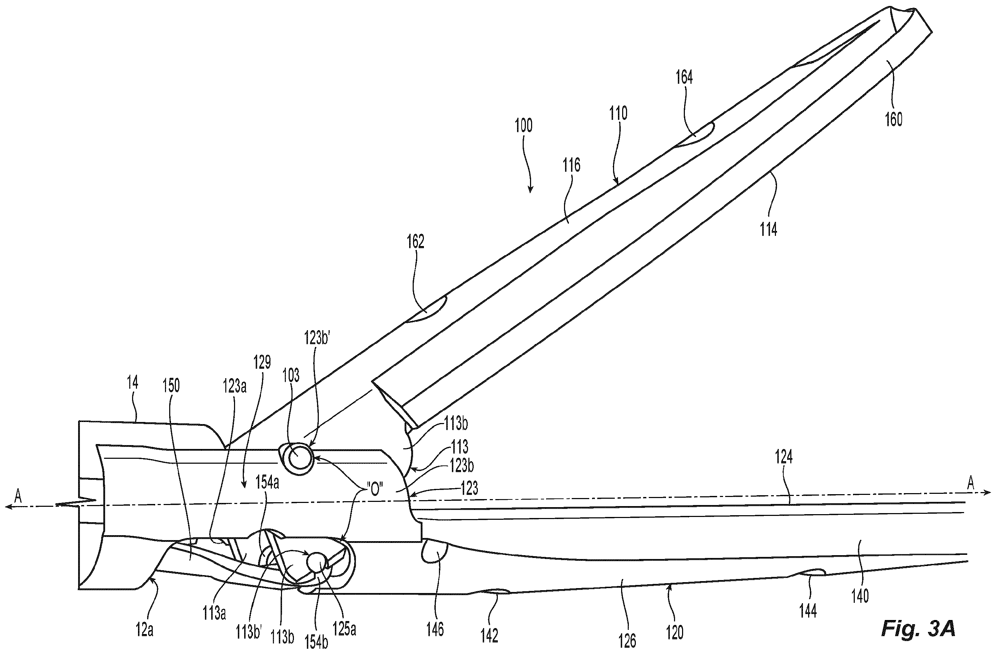

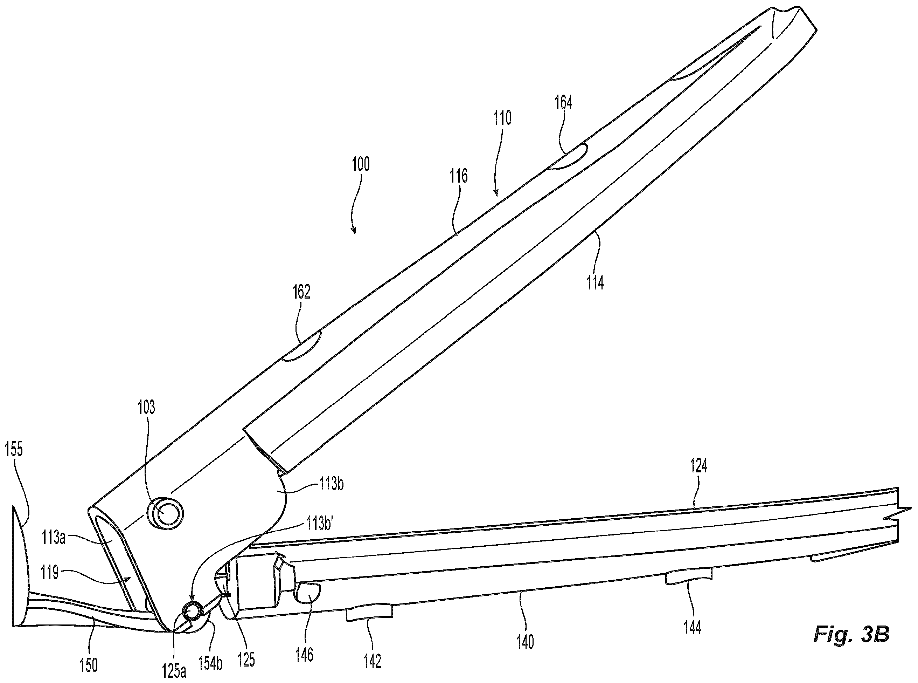

Forceps 10 includes a housing 20, a handle assembly 30, a trigger assembly 60, a rotating assembly 70, a first activation switch 80, a second activation switch 90, and an end effector assembly 100. Forceps 10 further includes a shaft 12 having a distal end portion 14 configured to (directly or indirectly) engage end effector assembly 100 and a proximal end portion 16 that (directly or indirectly) engages housing 20. Forceps 10 also includes cable "C" that connects forceps 10 to an energy source, e.g., an electrosurgical generator "G." Cable "C" includes a wire (or wires) (not shown) extending therethrough that has sufficient length to extend through shaft 12 in order to connect to one or both tissue-treating surfaces 114, 124 of jaw members 110, 120, respectively, of end effector assembly 100 (see FIGS. 3A and 3B) to provide energy thereto. First activation switch 80 is coupled to tissue-treating surfaces 114, 124 (FIGS. 1, 3A and 3B) and the electrosurgical generator "G" for enabling the selective activation of the supply of energy to jaw members 110, 120 for treating, e.g., cauterizing, coagulating/ desiccating, and/or sealing, tissue. Second activation switch 90 is coupled to thermal cutting element (not shown) of jaw member 120 and the electrosurgical generator "G" for enabling the selective activation of the supply of energy to thermal cutting element 150 for thermally cutting tissue. Details relating to various envisioned thermal cutting elements are disclosed in commonly-owned U.S. Patent Application Serial No. 63/073,397.

Handle assembly 30 of forceps 10 includes a fixed handle 50 and a movable handle 40. Fixed handle 50 is integrally associated with housing 20 and handle 40 is movable relative to fixed handle 50. Movable handle 40 of handle assembly 30 is operably coupled to a drive assembly 170 (shown generally in phantom) that, together, mechanically cooperate to impart movement of one or both of jaw members 110, 120 of end effector assembly 100 about a pivot 103 between a spaced-apart position and an approximated position to grasp tissue between tissue-treating surfaces 114, 124 of jaw members 110, 120. As shown in FIG. 1, movable handle 40 is initially spaced-apart from fixed handle 50 and, correspondingly, jaw members 110, 120 of end effector assembly 100 are disposed in the spaced-apart position. Movable handle 40 is depressible from this initial position to a depressed position corresponding to the approximated position of jaw members 110, 120. Rotating assembly 70 includes a rotation wheel 72 that is selectively rotatable in either direction to correspondingly rotate end effector assembly 100 relative to housing 20.

Various drive assemblies 170 are envisioned such as those described in U.S. Patent Nos. 10,953,757 and 9,113,903. The various envisioned drive assemblies 170 are configured to translate a drive member 150 relative to the distal end 14 of the shaft 12 to actuate the jaw members 110, 120 between open and closed positions (FIGS. 3A and 3B). Unilateral and bilateral jaw members 110, 120 are contemplated.

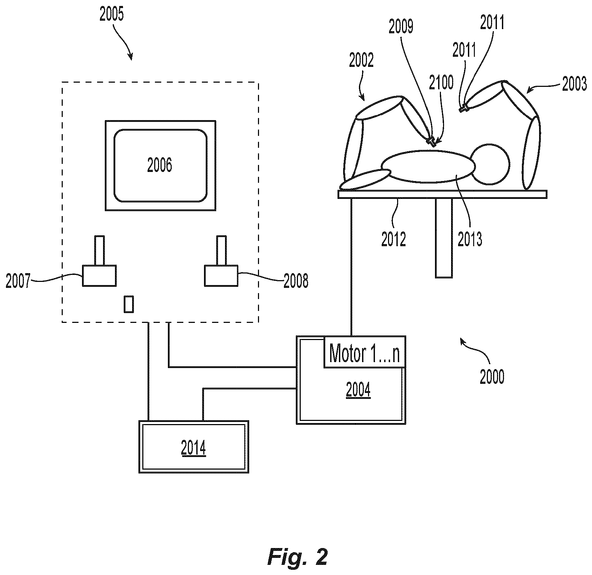

Referring to FIG. 2, a robotic surgical instrument provided in accordance with the present disclosure is shown generally identified by reference numeral 2000. Aspects and features of robotic surgical instrument 2000 not germane to the understanding of the present disclosure are omitted to avoid obscuring the aspects and features of the present disclosure in unnecessary detail.

Robotic surgical instrument 2000 includes a plurality of robot arms 2002, 2003; a control device 2004; and an operating console 2005 coupled with control device 2004. Operating console 2005 may include a display device 2006, which may be set up in particular to display three-dimensional images; and manual input devices 2007, 2008, by means of which a surgeon may be able to telemanipulate robot arms 2002, 2003 in a first operating mode. Robotic surgical instrument 2000 may be configured for use on a patient 2013 lying on a patient table 2012 to be treated in a minimally invasive manner. Robotic surgical instrument 2000 may further include a database 21014, in particular coupled to control device 2004, in which are stored, for example, pre-operative data from patient 2013 and/or anatomical atlases.

Each of the robot arms 2002, 2003 may include a plurality of members, which are connected through joints, and an attaching device 2009, 2011, to which may be attached, for example, an end effector assembly 2100, 2200, respectively. End effector assembly 2100 is similar to end effector assembly 100 (FIGS. 3A and 3B), although other suitable end effector assemblies for coupling to attaching device 2009 are also contemplated. End effector assembly 2200 may be any end effector assembly, e.g., an endoscopic camera, other surgical tool, etc. Robot arms 2002, 2003 and end effector assemblies 2100, 2200 may be driven by electric drives, e.g., motors, that are connected to control device 2004. Control device 2004 (e.g., a computer) may be configured to activate the motors, in particular by means of a computer program, in such a way that robot arms 2002, 2003, their attaching devices 2009, 2011, and end effector assemblies 2100, 2200 execute a desired movement and/or function according to a corresponding input from manual input devices 2007, 2008, respectively. Control device 2004 may also be configured in such a way that it regulates the movement of robot arms 2002, 2003 and/or of the motors.

Turning to FIG. 3A and 3B, one embodiment of a known end effector assembly 100, as noted above, includes first and second jaw members 110, 120. Each jaw member 110, 120 may include a structural support 160, 140, a jaw housing 116, 126, and a tissue-treating surface 114, 124, respectively. Alternatively, only one of the jaw members, e.g., jaw member 120, may include the structural support 140, jaw housing 126, and tissue-treating surface 124. In such embodiments, the other jaw member, e.g., jaw member 110, may be formed as a single unitary body, e.g., a piece of conductive material acting as the structural support 160 and jaw housing 116 and defining the tissue-treating surface 114. An outer surface of the jaw housing 116, in such embodiments, may be at least partially coated with an insulative material or may remain exposed. Tissue-treating surfaces 114, 124 may be pre-formed and engaged with jaw housings 116, 126 via overmolding, adhesion, mechanical engagement, etc. The structural supports 160, 140 for the jaw members 110, 120 may also be engaged to the respective jaw housings 116, 126 in a similar fashion via overmolding, adhesion, mechanical engagement, etc. as explained in more detail below.

As shown in FIGS. 3A and 3B, jaw members 110 and 120 are pivotably supported for rotation about pivot 103 and may be unilateral or bilateral depending upon a particular purpose. Outer housing 116 of jaw member 110 is configured to mechanically engage structural support 160 via one or more detents 162, 164 in a snap-fit manner or during an overmolding step. Jaw member 110 also includes a U-shaped proximal flange 113 having sides 113a and 113b that cooperatively define a cuff 119 configured to receive the drive member 150 as explained below. The sides 113a, 113b of flange 113 define opposing pivot bar cradles 113b' (other cradle defined in side 113a not shown) therein configured to receive a pivot bar 125a operably associated therewith.

Pivot bar 125a forms part of (or is welded, crimped or riveted or otherwise secured to) the respective cradles 113b' of sides 113a, 113b of the proximal flange 113 during a manufacturing step. The distal end of the drive member 150 is secured (or otherwise engaged) the pivot bar 125a while the opposite end of the drive member 150 is secured to or operably associated with the drive tube 155 slidably disposed within a bore 12a defined through shaft 12. Upon actuation of the handle 40, drive assembly 170 translate the drive tube 155. The distal end of the drive member 150 may be rotatably secured to the pivot bar 125a in a snap-fit or other manner to facilitate smooth operation of the mechanical coupling.

Jaw member 120 includes outer housing 126 configured to mechanically engage structural support 140 via one or more detents 142, 144 in a snap-fit manner or during an overmolding step. Jaw member 120 also includes a U-shaped proximal flange 123 having sides 123a and 123b that cooperatively define a cuff 129 configured to receive the proximal flange 113 of jaw member 110 as explained below. Flanges 123 and 113 may additionally act as tissue stops. The sides 123a, 123b of flange 123 define opposing pivot cradles 123b' (other cradle defined in side 123a not shown) therein configured to receive the pivot 103 operably associated with jaw member 110 (FIG. 3A). Pivot 103 is disposed through sides 113a, 113b of flange 113 towards an upper end thereof and is configured to allow rotation of jaw member 110. The pivot 103 rotates within the cradles 123b' defined in sides 123a, 123b of proximal flange 123 of jaw member 120 upon translation of the drive member 150 and rotation of the jaw member 110.

The second jaw member 120 defines a longitudinal axis A-A therethrough, the pivot 103 being disposed on one side the longitudinal axis A-A and the pivot bar 125a on the other to form an offset "O". Cradles 123b' (and other cradle defined in side 123a - not shown) may be defined in an uppermost edges of the proximal flanges 123a, 123b to maximize the distance or offset "O" between the pivot 103 and the pivot bar 125a to maximize the mechanical advantage therebetween. It is contemplated that the offset "O" between the pivot 103 and the pivot bar 125a provides a substantially constant opening and closure force between the first and second jaw members 110, 120 through the range of motion therebetween.

Jaw member 120 is secured to the distal end 14 of shaft 12. By mounting the pivot 103 within cradle 123b' (and cradle of side 123a) and securing jaw member 120 to the end 14 of the shaft 12, the end effector assembly 100 is held secure relative to the shaft 12. Actuating the handle 40 to translate drive tube 155 and, in turn, drive member 150, will pivot jaw member 110 relative to jaw member 120 by virtue of pivot bar 125a camming within the sides 113a, 113b of the proximal flange 113 about pivot 103.

Securing the pivot bar 125a to the sides 113a, 113b of the proximal flange 113 which, in turn, is directly coupled to the drive tube 155, minimizes hysteresis between the various mechanical components allowing finer dissection capabilities and a more consistent jaw closure and opening force. Providing consistent jaw closure forces and resulting sealing pressures between jaw members 110, 120 produces more consistent sealing and subsequent cutting of tissue and facilitates dissection. Moreover, with near constant closure forces throughout the entire handle 40 stroke, opening and closing the jaw members 110, 120 during poke and spread type dissection, translates to a consistent force therebetween enhancing the overall feel of the instrument during use.

Further, the mechanical couplings, i.e., drive tube 155 to drive member 150 to pivot bar 125a to proximal flange 113, do not extend outside the overall dimensions of the outer periphery of the elongated shaft 12 which reduces the chances of catching tissue during use while also maximizing the area for knife translation if used with a mechanical knife (Not shown).

The offset design of the pivot bar 125a and the pivot 103 provides a large mechanical advantage when the jaw members 110, 120 are being approximated about tissue but less of a mechanical advantage when the jaw members 110, 120 are moved to a fully open position. However, over the stroke of the closure of the jaw members 110, 120, the offset mechanical arrangement of the pivot bar 125a and the pivot 103 will yield more consistent closure pressure against tissues of varying sizes. Moreover, the near constant opening and closure forces provide the user with a consistent feel when opening and closing the jaw members 110, 120 for fine dissection purposes.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. An end effector assembly (100) for an electrosurgical instrument, comprising:
a first jaw member (110) having a jaw housing (116) supporting an electrically conductive tissue engaging surface (114) thereon, the jaw housing including a U-shaped proximal flange (113) having opposing sides (113a, 113b) defining a cuff (119) therebetween, each side including a cradle (113a', 113b') defined therein configured to secure a pivot bar (125a) therein, the proximal flange configured to operably support a pivot (103) therein;
a second jaw member (120) having a jaw housing (126) supporting an electrically conductive tissue engaging surface 124) thereon in opposition to the tissue engaging surface of the first jaw member. the jaw housing including a U-shaped proximal flange (123) having opposing sides (123a, 123b) defining a cuff (129) therebetween configured to receive the proximal flange of the first jaw member, each side of the second proximal flange including a cradle (123a, 123b) defined therein configured to operably support the pivot; and
a drive member (150) operably secured to the pivot bar wherein actuation the drive member translates the pivot bar to rotate the first jaw member relative to the second jaw member about the pivot wherein the second jaw member defines a longitudinal axis (A-A) therethrough, **characterised by**
the pivot being disposed on one side of the longitudinal axis and the pivot bar being offset from the pivot on the other side of the longitudinal axis wherein the cradle of the second jaw member is defined in an upper edge of the proximal flange thereof maximizing the offset between the pivot and the pivot bar increasing mechanical advantage therebetween.

2. The end effector assembly according to claim 1, wherein the pivot bar is welded, crimped or riveted to the cradle of each side of the proximal flange of the first jaw member.

3. The end effector assembly according to claim 1 or 2, wherein the first jaw member moves relative the second jaw member upon actuation of the drive member.

4. The end effector assembly according to claim 1, wherein the offset between the pivot and the pivot bar provides a substantially constant closure force between the first and second jaw members through the range of motion therebetween.

5. The end effector assembly according to claim 4, wherein the offset between the pivot and the pivot bar provides a substantially constant opening and closing force between the first and second jaw members through the range of motion therebetween.

6. An electrosurgical instrument, comprising:
a housing including a handle and an elongated shaft extending therefrom supporting and end effector assembly as claimed in any preceding claim at a distal end thereof, and
a drive tube selectively translatable within the elongated shaft, the drive tube including the drive member disposed at a distal end thereof.

7. The electrosurgical instrument according to any preceding claim, wherein the elongated shaft defines an outer periphery and wherein the proximal flanges of the first and second jaw members remain inside the outer periphery during the range of motion between jaw members.

## Patentansprüche

1. Endeffektoranordnung (100) für ein elektrochirurgisches Instrument, die Folgendes umfasst:
ein erstes Backenelement (110) mit einem Backengehäuse (116), das eine elektrisch leitfähige Gewebeeingriffsfläche (114) darauf trägt, wobei das Backengehäuse einen U-förmigen proximalen Flansch (113) mit gegenüberliegenden Seiten (113a, 113b) umfasst, die eine Manschette (119) dazwischen definieren, wobei jede Seite eine darin definierte Halterung (113a', 113b') umfasst, die dazu ausgelegt ist, eine Schwenkstange (125a) darin zu sichern, wobei der proximale Flansch dazu ausgelegt ist, einen Drehzapfen (103) funktionsfähig darin zu tragen;
ein zweites Backenelement (120) mit einem Backengehäuse (126), das eine elektrisch leitfähige Gewebeeingriffsfläche (124) gegenüber der Gewebeeingriffsfläche des ersten Backenelements darauf trägt, wobei das Backengehäuse einen U-förmigen proximalen Flansch (123) mit gegenüberliegenden Seiten (123a, 123b) umfasst, die eine Manschette (129) dazwischen definieren, die dazu ausgelegt ist, den proximalen Flansch des ersten Backenelements aufzunehmen, wobei jede Seite des zweiten proximalen Flansches eine darin definierte Halterung (123a, 123b) umfasst, die dazu ausgelegt ist, den Drehzapfen funktionsfähig zu tragen; und
ein Antriebselement (150), das funktionsfähig an der Schwenkstange gesichert ist, wobei eine Betätigung des Antriebselements die Schwenkstange verschiebt, um das erste Backenelement relativ zu dem zweiten Backenelement um den Drehpunkt zu drehen
wobei das zweite Backenelement eine Längsachse (A-A) dort hindurch definiert, **dadurch gekennzeichnet, dass** der Drehzapfen auf einer Seite der Längsachse angeordnet ist und die Schwenkstange von dem Drehzapfen auf der anderen Seite der Längsachse versetzt ist, wobei das Halterung des zweiten Backenelements in einer oberen Kante des proximalen Flansches davon definiert ist, wodurch der Versatz zwischen dem Drehzapfen und der Schwenkstange maximiert wird, wodurch der Kraftgewinn dazwischen erhöht wird.

2. Endeffektoranordnung nach Anspruch 1, wobei die Schwenkstange an die Halterung jeder Seite des proximalen Flansches des ersten Backenelements geschweißt, gequetscht oder genietet ist.

3. Endeffektoranordnung nach Anspruch 1 oder 2, wobei sich das erste Backenelement bei Betätigung des Antriebselements relativ zu dem zweiten Backenelement bewegt.

4. Endeffektoranordnung nach Anspruch 1, wobei der Versatz zwischen dem Drehzapfen und der Schwenkstange eine im Wesentlichen konstante Schließkraft zwischen dem ersten und dem zweiten Backenelement über den Bewegungsbereich dazwischen bereitstellt.

5. Endeffektoranordnung nach Anspruch 4, wobei der Versatz zwischen dem Drehzapfen und der Schwenkstange eine im Wesentlichen konstante Öffnungs- und Schließkraft zwischen dem ersten und dem zweiten Backenelement über den Bewegungsbereich dazwischen bereitstellt.

6. Elektrochirurgisches Instrument, das Folgendes umfasst:
ein Gehäuse, das einen Griff und einen sich davon erstreckenden länglichen Schaft umfasst, der eine Endeffektoranordnung nach einem der vorhergehenden Ansprüche an einem distalen Ende davon trägt, und
ein Antriebsrohr, das selektiv innerhalb des länglichen Schafts verschiebbar ist, wobei das Antriebsrohr das Antriebselement umfasst, das an einem distalen Ende davon angeordnet ist.

7. Elektrochirurgisches Instrument nach einem der vorhergehenden Ansprüche, wobei der längliche Schaft einen Außenumfang definiert und wobei die proximalen Flansche des ersten und des zweiten Backenelements während des Bewegungsbereichs zwischen Backenelementen innerhalb des Außenumfangs verbleiben.

## Revendications

1. Ensemble effecteur terminal (100) pour instrument électrochirurgical, comprenant :
un premier élément formant mâchoire (110) présentant un corps de mâchoire (116) supportant une surface de contact avec un tissu électroconductrice (114), le corps de mâchoire comprenant une saillie proximale en U (113) présentant des côtés opposés (113a, 113b) définissant un manchon (119) entre eux, chaque côté comprenant une échancrure (113a', 113b') définie dans celui-ci configurée pour y fixer une barre de pivotement (125a), la saillie proximale étant configurée pour supporter de manière fonctionnelle un pivot (103) dans celle-ci ;
un second élément formant mâchoire (120) présentant un corps de mâchoire (126) supportant une surface de contact avec un tissu électroconductrice (124) en opposition à la surface de contact avec un tissu du premier élément formant mâchoire, le corps de mâchoire comprenant une saillie proximale en U (123) présentant des côtés opposés (123a, 123b) définissant entre eux un manchon (129) configuré pour recevoir la saillie proximale du premier élément formant mâchoire, chaque côté de la seconde saillie proximale comprenant une échancrure (123a, 123b) définie dans celui-ci configurée pour supporter de manière fonctionnelle le pivot ; et
un élément d'entraînement (150) fixé de manière fonctionnelle à la barre de pivotement, l'actionnement de l'élément d'entraînement déplaçant par translation la barre de pivotement afin de faire tourner le premier élément formant mâchoire par rapport au second élément formant mâchoire autour du pivot
le second élément formant mâchoire définissant un axe longitudinal (A-A) le traversant, **caractérisé en ce que** le pivot est disposé d'un côté de l'axe longitudinal et la barre de pivotement est décalée relativement au pivot de l'autre côté de l'axe longitudinal, l'échancrure du second élément formant mâchoire étant définie dans un bord supérieur de la saillie proximale de celui-ci, maximisant ainsi le décalage entre le pivot et la barre de pivotement, ce qui augmente l'avantage mécanique entre eux.

2. Ensemble effecteur terminal selon la revendication 1, dans lequel la barre de pivotement est soudée, sertie ou rivetée à l'échancrure de chaque côté de la saillie proximale du premier élément formant mâchoire.

3. Ensemble effecteur terminal selon la revendication 1 ou 2, dans lequel le premier élément formant mâchoire se déplace par rapport au second élément formant mâchoire à la suite de l'actionnement de l'élément d'entraînement.

4. Ensemble effecteur terminal selon la revendication 1, dans lequel le décalage entre le pivot et la barre de pivotement produit une force de fermeture sensiblement constante entre les premier et second éléments formant mâchoires sur la plage de mouvement entre eux.

5. Ensemble effecteur terminal selon la revendication 4, dans lequel le décalage entre le pivot et la barre de pivotement produit une force d'ouverture et de fermeture sensiblement constante entre les premier et second éléments formant mâchoires sur la plage de mouvement entre eux.

6. Instrument électrochirurgical comprenant :
un corps comprenant une poignée et une tige allongée s'étendant à partir de celle-ci supportant un ensemble effecteur terminal selon l'une quelconque des revendications précédentes à une extrémité distale de celle-ci, et
un tube d'entraînement pouvant être déplacé sélectivement par translation à l'intérieur de la tige allongée, le tube d'entraînement comprenant l'élément d'entraînement disposé à une extrémité distale de celui-ci.

7. Instrument électrochirurgical selon l'une quelconque des revendications précédentes, dans lequel la tige allongée définit une périphérie extérieure et dans lequel les saillies proximales des premier et second éléments formant mâchoires restent à l'intérieur de la périphérie extérieure au sein de la plage de mouvement entre les éléments formant mâchoires.
